# EUROPEAN PATENT SPECIFICATION

(11) **EP 4 147 732 B1**
(45) Date of publication and mention of the grant of the patent: **26.02.2025**
(21) Application number: 21798935.9
(22) Date of filing: 03.05.2021
(51) Int. Cl.: A61M 1/00, A61F 13/00

(54) **DRESSING FOR NEGATIVE-PRESSURE THERAPIES**
VERBAND FÜR UNTERDRUCKTHERAPIEN
PANSEMENT POUR THÉRAPIES PAR PRESSION NÉGATIVE

(30) Priority: 06.05.2020 ES 202030820 U
(43) Date of publication of application: 15.03.2023
(73) Proprietor: Servicio Cántabro de Salud, 39011 Santander (Cantabria) (ES)
(72) Inventor: CASTILLO SUESCÚN, Federico, 39011 Santander (ES)
(74) Representative: Herrero & Asociados, S.L.
(86) International application number: PCT/ES2021/070302
(87) International publication number: WO 2021/224526

(56) References cited:
- EP-A1- 3 305 957
- WO-A1-2019/234273
- WO-A1-95/25482
- CN-A- 107 693 213
- GB-A- 2 104 558
- US-A- 4 207 885
- US-A1- 2012 226 214
- US-A1- 2020 139 025

## Description

### OBJECT OF THE INVENTION

The present invention falls within the technical field of dressings, more specifically in the field of dressings specially designed for the thorax, abdomen and extremities, as well as in the field of suction or pumping devices for medical use and refers in particular to a dressing for the application of negative pressure therapies.

### BACKGROUND OF THE INVENTION

Within the technical field of surgery, negative pressure therapy (hereafter referred to as NPWT) consists of the application of sub-atmospheric pressure to the wound bed as a form of topical, non-invasive wound treatment, facilitating healing through a multimodal action. It is a widely used option that provides a number of benefits to facilitate healing, including reduced wound retraction, removal of exudate and non-viable tissue, improved blood supply, promotion of granulation tissue formation and, finally, physical stimulation of cell mitosis.

NPWT can be broadly divided into three main groupings. The first, known as NPWT in the open abdomen (OA), was designed with the management of abdominal compartment syndrome (ACS) in mind. Damage control surgery, trauma and ACS are common reasons for leaving the OAS. However, surveys such as the International Register of Open Abdomen (IROA) now show that septic problems such as peritonitis and pancreatitis account for more than 50% of the use of OA in the first world.

A key point to optimize outcome is early abdominal closure within 7 days, as failure to do so will increase morbidity, mortality and bowel fistula formation. Temporary abdominal closure (TAC) protects the viscera and drains fluids as part of the treatment of ACS, but the key component of this therapy in OA is the NPWT-associated visceral protection lamina (VPL), which is not present in the other types of NPWT.

VPLs were designed in the various commercial brands to actively remove abdominal fluid and help reduce oedema but have difficulty removing high viscosity fluid and particulate drainage such as that seen in abdominal septic processes and pancreatitis. This drainage, called complex exudate, is a protein-rich fluid that can coagulate in the VPL, and also contains blood, purulent material and various solid and semi-solid particles from clots, fibrin and necrosis removed from tissues such as the pancreas, These accumulate in the device, mainly in the VPL, quickly saturate it and obstruct it, making it difficult to control the septic focus and oedema, prolonging the time of use of the NPWT, which increases the feared complications of OA such as intestinal fistula.

The Spanish utility model with publication number ES1215290, owned by this applicant, describes a multilayer visceral protection dressing incorporating an intermediate layer comprising a plurality of evenly distributed alveoli or bubbles filled with low-pressure air. This multilayer structure optimizes the distribution of negative pressure across the surface of the sheet to the most distal areas and improves the drainage capacity of complex exudate and reduces clogging of the device. It also produces a uniform distribution of the negative pressure over the entire surface in contact with the VPL, however, it may fail in case of having to drain or communicate with the negative pressure source collections, abscesses in sites far from the VPL and difficult to access as for example the omentum transcavity in pancreatitis or an infected collection or isolated abscess such as a retrocolic abscess in a perforated diverticulitis. In these cases, for anatomical reasons, the VPL design may not be effective and may require an extra device to allow better drainage of the cavity and early control of the septic focus.

In the case of chronic wounds secondary to unhealed surgical wounds, traumatic injuries, venous ulcers, pressure ulcers, diabetic foot ulcers, which may be associated with infection, are having a devastating impact on health services in terms of associated complications and expenditure of healthcare resources, in addition to the deterioration in the quality of life of increasingly long-lived patients. In the last 30 years, the incidence of diabetes mellitus has almost quadrupled, affecting more than 422 million adults worldwide.

The time to complete healing-epithelialization varies according to some factors and comorbidities such as diabetes, autoimmune diseases, peripheral vascular disease, obesity, anatomical location of the lesion and medications. However, wound healing will largely depend on the condition of the wound bed, vascular supply, microcirculation, presence of infection and bacterial load.

Several NPWT application systems are currently known for this particular type of wound, as well as specific dressings and drainage components, all based on polyurethane or polyvinyl foam in direct contact with the wound. These foams produce rapid saturation in case of complicated exudate, infection and/or necrosis.

Polyurethane foam is used in multiple commercial devices, but all focus on some small design changes. It is an open pore foam (400-600 microns) that changes structure with negative pressure, which significantly decreases pore size and in wounds with abundant debris, necrosis and infection does not provide an even distribution of vacuum therapy across the wound surface. A phenomenon of foam saturation has also been identified which decreases the effectiveness of the therapy and increases usage times and costs.

Finally, in the case of surgical wounds where infection prevention is sought, and in light and superficial wounds that do not have a lot of exudates, disposable NPWT systems are used to help control low to moderate exudate. These generally consist of a small portable pump without a reservoir, capable of generating a lower negative pressure than conventional devices, as described in Spanish patent publication number ES2555493T3. The pump is connected to a dressing containing an absorbent material composed of cellulose fibres that traps exudate but quickly becomes saturated. Excess exudate can flow through the tube that conducts the negative pressure in the inlet direction into the vacuum pump which, not having a reservoir, is damaged.

Other, also disposable NPWT systems, in which the pump differs in that the pump lasts 30 days, are connected to a dressing in which the layer in direct contact with the wound consists of Sodium Carboxymethicellulose, which gels on contact with the exudate. This is a disadvantage because the gel hinders and even interrupts the distribution of negative pressure.

As can be seen, both dressings present a similar problem, based on rapid saturation and clogging by viscous contents in a real wound, making them of little use in infected wounds. Although they are intended to highlight the benefit of helping to reduce the likelihood of infection in surgical incisions, preferably abdominal, they cease to work in the short term, increasing the cost of therapy. In addition, the high environmental impact of these disposable pumps must be considered.

In view of the state of the art, there is therefore a need for a specific dressing for negative pressure therapy for this type of surgical incisions and mild to moderate wounds to avoid the problems described above.

### DESCRIPTION OF THE INVENTION

The subject matter of the invention consists of a dressing for negative pressure therapies comprising a plurality of overlapping laminar layers solidly linked to each other by at least their respective perimeter edges. In the most preferred embodiments, the dressing comprises from two to four layers in total.

Each of these layers has properties and capabilities, including air or other gas-filled housings such as nitrogen or CO₂, preferably at low pressure, as well as drainage channels and fluid perfusion channels.

The abdominal viscera protection dressing described above is temporarily placed over an open wound. A sponge body is placed on top of the dressing, on which a negative pressure therapy system is supported.

By applying negative pressure to the dressing, the air-filled pockets prevent the sheets from sticking together in the first place, as this configuration prevents rapid saturation and clogging of the polyurethane sponge by other dressings with more viscous and solid elements such as pus debris, fibrin, clots and necrosis.

The air pockets also prevent adherence to internal organ tissues, and internal organ effluents (fluids, necrotic tissues, clots, etc.) are drained homogeneously through the through holes. The uniform distribution of both air pockets and through holes over the entire surface of the respective layers optimizes the drainage capacity and the distribution of the negative pressure therapy, allowing it to be exerted even in the most distal areas of the cavity.

The dressing is preferably made of a material selected from the group of EVAS, PU, PVC, PE, PET, PTFE, TPE, silicone or a mixture thereof. Furthermore, the dressing can be adjusted in size, depending on the required treatment, by cutting out the necessary surface area.

Among other advantages, the negative pressure therapy dressing thus described provides optimized drainage of exudates, enables debridement of necrotic tissue and offers the possibility of instillation or perfusion for easier diffusion of the instilled or perfused fluid to the wound surface.

The various preferred embodiments of the dressing are able to solve the problems described above. Thus, in the case of NPWT applied in the open abdomen (OA), the dressing can help drain deep or distant areas in the abdomen. It can also be used folded on itself, in the form of a temporary packing to perform site-specific negative pressure limitation by removing or blocking the interlayer with the gaseous lodgments.

In the case of chronic wounds, it must first be considered that these wounds come in a wide variety of shapes and sizes, so the dressings used in NPWT must be able to adapt to these morphologies. As mentioned above, polyurethane foam is the key component of current NPWT dressings for these wounds.

In the embodiments designed for these chronic wounds, the dressing comprises sheets made of polyurethane foam and an element for uniform pressure distribution. In a first embodiment, this element consists of a cylinder made of a fenestrated polyurethane sheet folded on itself, so that the alveoli are arranged in a tubular shape towards the outside, and distal orifices forming a central channel. The combination of both elements allows the administration of a fluid from the NPWT machine, hitherto used for instillation.

In a second embodiment, the internal element consists of an intermediate sheet, also made of polyurethane foam, provided with holes in contact with the wound, so that a network of channels is generated that allows the uniform transmission of the negative pressure and also the infusion of fluids from the NPWT machine.

For chronic wounds located in complex sites, such as the interdigital spaces of the feet, the knitted fabric is reinforced with a bearded thread sewn in a spring form. Currently, materials such as gauze or foam are used for such wounds, which are very difficult to apply and become dislodged with use.

Because of the anatomical complication of placing the therapy in these distal sites, the bearded spring fabric and encapsulated sheet allow for a more comfortable and adapted placement and also allows for an extension of the NP source so that it is not in the same place.

### DESCRIPTION OF THE DRAWINGS

In order to complement the description being made and in order to assist in a better understanding of the features of the invention, in accordance with a preferred example of a practical embodiment thereof, a set of drawings is attached hereto as an integral part of the said description, in which the following is illustratively and non-limitingly depicted:
Figure 1.- Shows a perspective exploded view of a first embodiment of the dressing.
Figure 2.- Shows a side view of a longitudinal cut of the first embodiment of the dressing, showing its main constituent elements.
Figure 3.- Shows a perspective exploded view of a second embodiment of the dressing.
Figure 4.- Shows a side view of a longitudinal cut of the second embodiment of the dressing.
Figure 5.- Shows a side view of a longitudinal cut of the third embodiment of the dressing.
Figure 6.- Shows a perspective exploded view of a third embodiment of the dressing.
Figure 7.- Shows a side view of a longitudinal cut of the third embodiment of the dressing.
Figure 8.- Shows a perspective view of the third embodiment of the dressing, configured as a capsule.

### PREFERRED EMBODIMENT OF THE INVENTION

A detailed explanation of an example of a preferred embodiment of the subject matter of the present invention is given below with the aid of the figures referred to above.

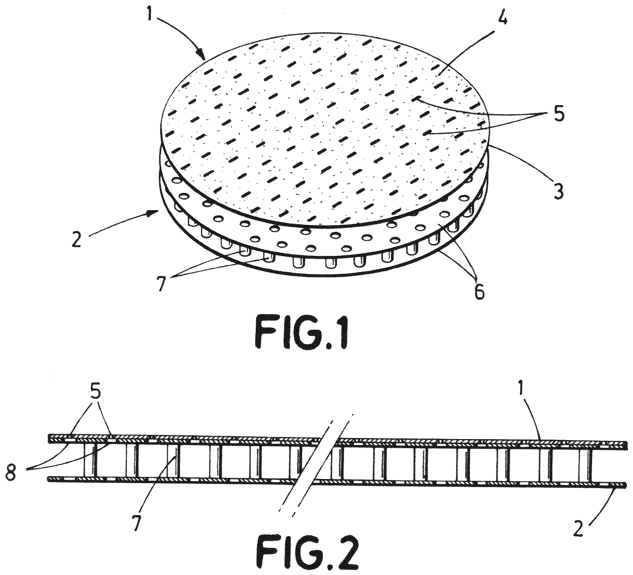

The dressing for negative pressure therapy described is made up of a multilayer structure, which in its first preferred embodiment comprises a first layer (1) and a second layer (2), linked together by their respective perimeter edges, as shown in figure 1.

The first layer (1) consists of a laminar body with a first inner face (3), which is intended to face inwards and face the second layer (2), and a first outer face (4), which is intended to face outwards. The laminar body also comprises a number of first through holes (5) uniformly distributed, which allow the passage of fluid through it, giving the first layer (1) a fenestrated character that allows an adequate distribution of a negative pressure.

The second layer (2) has geometry and dimensions essentially similar to those of the first layer (1) and is made up of two lamellar bodies (6) solidly linked together by means of a plurality of tubular elements (7) uniformly distributed and inside which a gaseous fluid is housed and maintained at low pressure. The distribution of the tubular elements (7) is such that the mutual separation distance between two adjacent tubular elements (7) is equal to or greater than half their diametrical dimensions. In this first preferred embodiment, the tubular elements (7), of essentially cylindrical geometry, are linked perpendicularly at each of their ends to the respective lamellar bodies (6).

Each of the lamellar bodies (6) incorporates a plurality of second through holes (8) that allow the passage of fluid through them, giving the second layer (2) a fenestrated character that contributes to allow an adequate distribution of negative pressure. Said second through holes (8), which in this preferred embodiment have an essentially rhomboidal geometry, are evenly distributed in each laminar body (6) and so that they are adjacent to, but not coincident with, the inserts of the tubular elements, as illustrated in the detail of figure 2.

Figure 2 also shows that the first layer (1) and the second layer (2) overlap each other so that the first (5) and second through holes (8) face each other, creating channels that allow the passage of negative pressure between two opposite sides of the dressing.

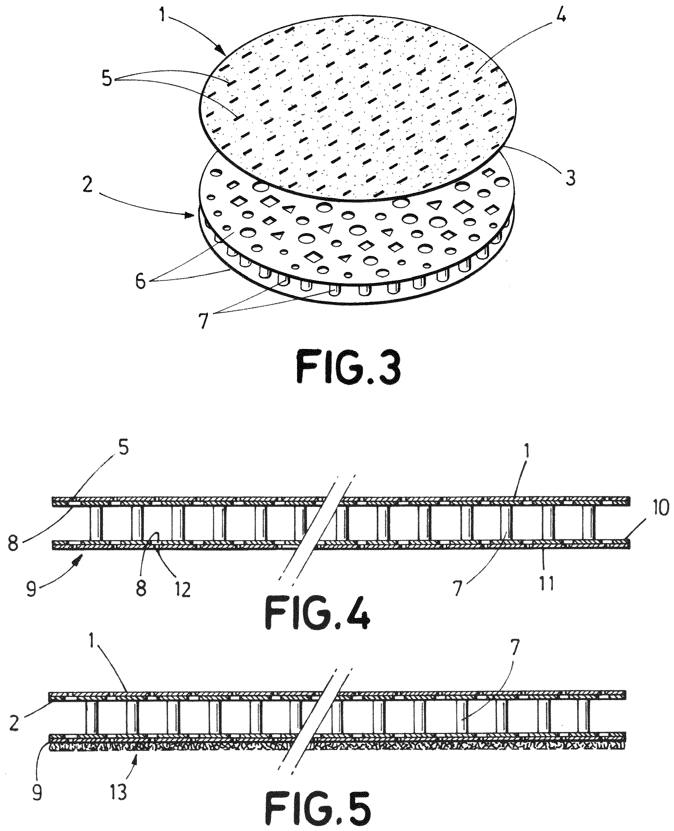

In a second preferred embodiment, illustrated in figures 3 and 4, the dressing additionally comprises a third layer (9) of geometry and dimensions essentially similar to those of the first layer (1) and the second layer (2). This second preferred embodiment is intended for application to light and superficial wounds and for protection of abdominal viscera.

The third layer (9) is made up of a laminar body with a second inner face (10), which is intended to face inwards and face the second layer (2), and a second outer face (11). The laminar body also comprises a plurality of third through holes (12) uniformly distributed, which allow the passage of fluid through it, giving the third layer (9) a fenestrated character that allows an adequate distribution of a negative pressure.

Figure 3 also shows that the first layer (1), the second layer (2) and the third layer (9) overlap each other so that the first (5), second (8) and third through holes (12) face each other, creating channels that allow the passage of the negative pressure between two opposite sides of the dressing. The second through holes (8) have different geometries and dimensions, as shown in figure 3.

In a third preferred embodiment, illustrated in figure 5, the dressing additionally comprises a fourth layer (13), with geometry and dimensions essentially similar to those of the first (1), second (2) and third (9) layers. In this third preferred embodiment, the fourth layer (13) allows the dressing to function for wounds with infection and necrosis.

In the third embodiment, the fourth layer (13) consists of a knitted fabric reinforced with a barbed thread sewn in the form of a spring, so as to allow the passage of fluids into the through holes (5), (8), (12). The fourth layer (13) thus described has a certain resistance to deformation exerted by the application of negative pressure. In this third preferred embodiment, the fourth layer (13) is made of polypropylene and is less than 8 mm thick.

In an example use of this third preferred embodiment, the fourth layer (13) is placed directly over an infected wound, such that the first layer (1) is free to attach to a negative pressure therapy system. By applying negative pressure to the dressing, efficient and homogeneous drainage of wound fluids is achieved through the mesh of the fourth layer (13) and the through holes (5), (8), (12), as the tubular elements (7) allow the negative pressure therapy to be distributed in a homogeneous manner which also optimizes the drainage capacity.

Moreover, the bearded thread of the fourth layer (13) contributes to the maintenance of a fresh wound and the removal of necrotic tissue when the dressing is removed, facilitating faster and more efficient healing.

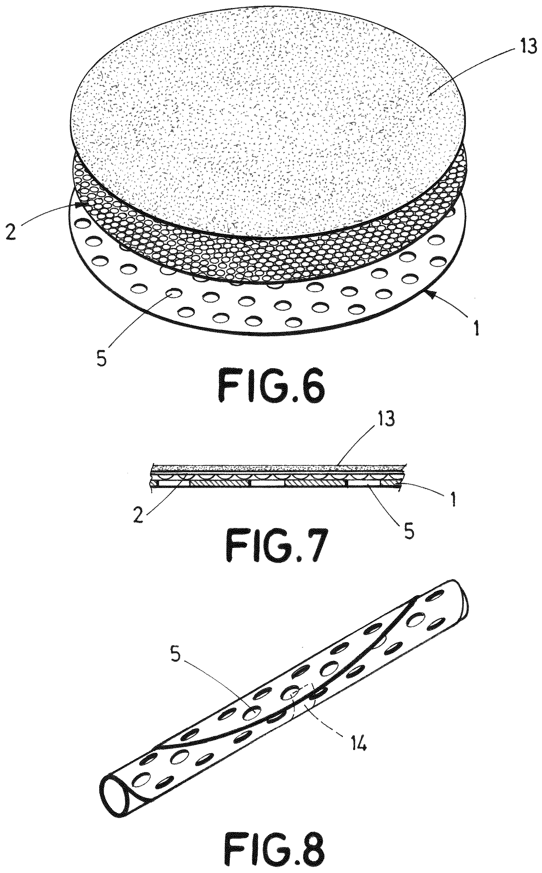

Figures 6, 7 and 8 show a fourth preferred embodiment of the dressing, in which the fourth layer (13) consists of a sheet of porous foamed material, in this case polyurethane foam. This preferred embodiment incorporates a closure element (14) designed to keep the dressing in a rolled and encapsulated configuration, as shown in figure 8.

This encapsulated configuration of the dressing allows it to be inserted and maintained in small wounds, such as those due to surgical incisions, where NPWT is required. The closure element (1) can be either an element external to the dressing, such as a small adhesive strip or a suture thread, or an element of its own, such as a die-cut or a tongue and groove joint.

## Claims

1. Dressing for negative pressure therapy comprising a multi-layer structure comprising a first layer (1) and a second layer (2), linked together by their respective perimeter edges, wherein the first layer (1) consists of a lamellar body comprising:
- a first inner face (3), intended to be inward facing and facing the second layer (2);
- a first outer face (4), intended to face outwards; and
- a plurality of first through holes (5) allowing fluid to pass through;
being the dressing **characterized in that** the second layer (2) comprises two lamellar bodies (6) solidly linked together, each of which comprises a plurality of second through holes (8) allowing the passage of fluid, and a plurality of tubular elements (7) arranged between the lamellar bodies (6), and inside which a pressurized gaseous fluid is housed and maintained,
wherein the first layer (1) and the second layer (2) overlap each other so that the first (5) and second through holes (8) face each other, creating channels that allow the passage of negative pressure between two opposite sides of the dressing.

2. Dressing according to claim 1, **characterized in that** it comprises a third layer (9) of geometry and dimensions essentially similar to those of the first layer (1) and the second layer (2), consisting of a lamellar body comprising:
- a second inner face (10), intended to face inwards and face the second layer (2);
- a second outer face (11); and
- a plurality of third through holes (12) allowing fluid to pass through;
wherein the first layer (1), second layer (2) and third layer (9) overlap each other so that the first (5), second (8) and third through holes (12) face each other, creating channels that allow negative pressure to pass between two opposite sides of the dressing.

3. Dressing according to claim 2 **characterized in that** it comprises a fourth layer (13) of geometry and dimensions essentially similar to those of the first (1), second (2) and third (9) layers.

4. Dressing according to claim 3, **characterized in that** the fourth layer (13) consists of a knitted fabric reinforced with a barbed thread and sewn in a spring-like manner, so as to allow the passage of fluids into the through holes (5), (8), (12).

5. Dressing according to claim 3 or 4 **characterized in that** the fourth layer (13) consists of a sheet of porous foamed material so as to allow the passage of fluids into the through holes (5), (8), (12).

6. Dressing according to claim 5 **characterized in that** it comprises a closure element (14) for holding the dressing in a rolled and encapsulated configuration.

7. Dressing according to any one of the preceding claims, **characterized in that** the tubular elements (7) have an essentially cylindrical geometry.

## Patentansprüche

1. Verband für die Unterdrucktherapie, umfassend eine mehrschichtige Struktur, die eine erste Schicht (1) und eine zweite Schicht (2) umfasst, die durch ihre jeweiligen Umfangsränder miteinander verbunden sind, wobei die erste Schicht (1) aus einem lamellenförmigen Körper besteht, der Folgendes umfasst
- eine erste Innenfläche (3), die dazu bestimmt ist, nach innen zu zeigen und der zweiten Schicht (2) zugewandt zu sein;
- eine erste Außenfläche (4), die dazu bestimmt ist, nach außen zu zeigen; und
- eine Mehrzahl von ersten Durchgangslöchern (5), die den Durchgang von Fluid ermöglichen;
wobei der Verband **dadurch gekennzeichnet ist, dass** die zweite Schicht (2) zwei lamellenförmige Körper (6) umfasst, die fest miteinander verbunden sind und von denen jeder eine Mehrzahl von zweiten Durchgangslöchern (8), die den Durchgang von Fluid ermöglichen, und eine Mehrzahl von röhrenförmigen Elementen (7) umfasst, die zwischen den lamellenförmigen Körpern (6) angeordnet sind und in denen ein unter Druck stehendes gasförmiges Fluid untergebracht und gehalten wird,
wobei die erste Schicht (1) und die zweite Schicht (2) einander überlappen, so dass die ersten (5) und zweiten Durchgangslöcher (8) einander zugewandt sind, wodurch Kanäle gebildet werden, die den Durchgang von Unterdruck zwischen zwei gegenüberliegenden Seiten des Verbandes ermöglichen.

2. Verband nach Anspruch 1, **dadurch gekennzeichnet, dass** er eine dritte Schicht (9) umfasst, deren Geometrie und Abmessungen im Wesentlichen ähnlich zu denen der ersten Schicht (1) und der zweiten Schicht (2) sind, und die aus einem lamellenförmigen Körper besteht, der Folgendes umfasst:
- eine zweite Innenfläche (10), die dazu bestimmt ist, nach innen zu zeigen und der zweiten Schicht (2) zugewandt zu sein;
- eine zweite Außenfläche (11); und
- eine Mehrzahl von dritten Durchgangslöchern (12), die den Durchgang von Fluid ermöglichen;
wobei die erste Schicht (1), die zweite Schicht (2) und die dritte Schicht (9) einander überlappen, so dass die ersten (5), zweiten (8) und dritten Durchgangslöcher (12) einander zugewandt sind, wodurch Kanäle gebildet werden, die den Durchgang von Unterdruck zwischen zwei gegenüberliegenden Seiten des Verbandes ermöglichen.

3. Verband nach Anspruch 2, **dadurch gekennzeichnet, dass** er eine vierte Schicht (13) umfasst, deren Geometrie und Abmessungen im Wesentlichen ähnlich zu denen der ersten (1), zweiten (2) und dritten (9) Schicht sind.

4. Verband nach Anspruch 3, **dadurch gekennzeichnet, dass** die vierte Schicht (13) aus einem mit einem Widerhakenfaden verstärkten und federartig vernähten Gestrick besteht, so dass der Durchtritt von Fluiden in die Durchgangslöcher (5), (8), (12) möglich ist.

5. Verband nach Anspruch 3 oder 4, **dadurch gekennzeichnet, dass** die vierte Schicht (13) aus einer Lage von porösem Schaumstoff besteht, so dass der Durchtritt von Fluiden in die Durchgangslöcher (5), (8), (12) möglich ist.

6. Verband nach Anspruch 5, **dadurch gekennzeichnet, dass** er ein Verschlusselement (14) umfasst, um den Verband in einer gerollten und eingekapselten Konfiguration zu halten.

7. Verband nach einem der vorhergehenden Ansprüche, **dadurch gekennzeichnet, dass** die röhrenförmigen Elemente (7) eine im Wesentlichen zylindrische Geometrie aufweisen.

## Revendications

1. Pansement pour thérapie par pression négative comprenant une structure multicouche comprenant une première couche (1) et une deuxième couche (2), reliées entre elles par leurs bords périphériques respectifs, dans lequel la première couche (1) consiste en un corps lamellaire comprenant :
- une première face interne (3), destinée à être tournée vers l'intérieur et faisant face à la deuxième couche (2) ;
- une première face externe (4), destinée à être tournée vers l'extérieur ; et
- une pluralité de premiers trous traversants (5) permettant le passage de fluide ;
le pansement étant **caractérisé en ce que** la seconde couche (2) comprend deux corps lamellaires (6) solidement liés entre eux, dont chacun comprend une pluralité de seconds trous traversants (8) permettant le passage de fluide, et une pluralité d'éléments tubulaires (7) disposés entre les corps lamellaires (6), et à l'intérieur desquels un fluide gazeux sous pression est logé et maintenu,
dans lequel la première couche (1) et la seconde couche (2) se chevauchent de sorte que les premiers (5) et seconds trous traversants (8) se font face, créant des canaux qui permettent le passage d'une pression négative entre deux côtés opposés du pansement.

2. Pansement selon la revendication 1, **caractérisé en ce qu'**il comprend une troisième couche (9) de géométrie et de dimensions essentiellement similaires à celles de la première couche (1) et de la deuxième couche (2), consistant en un corps lamellaire comprenant :
- une deuxième face interne (10), destinée à être tournée vers l'intérieur et face à la deuxième couche (2) ;
- une deuxième face externe (11) ; et
- une pluralité de troisièmes trous traversants (12) permettant le passage de fluide ; dans lequel la première couche (1), la deuxième couche (2) et la troisième couche (9) se chevauchent de sorte que les premiers (5), deuxièmes (8) et troisièmes trous traversants (12) se font face, créant des canaux qui permettent le passage d'une pression négative entre deux côtés opposés du pansement.

3. Pansement selon la revendication 2 **caractérisé en ce qu'**il comprend une quatrième couche (13) de géométrie et de dimensions essentiellement similaires à celles des première (1), deuxième (2) et troisième (9) couches.

4. Pansement selon la revendication 3, **caractérisé en ce que** la quatrième couche (13) consiste en un tissu tricoté renforcé par un fil barbelé et cousu à la manière d'un ressort, de manière à permettre le passage de fluides dans les trous traversants (5), (8), (12).

5. Pansement selon la revendication 3 ou 4 **caractérisé en ce que** la quatrième couche (13) consiste en une feuille de matériau expansé poreux de manière à permettre le passage de fluides dans les trous traversants (5), (8), (12).

6. Pansement selon la revendication 5 **caractérisé en ce qu'**il comprend un élément de fermeture (14) pour maintenir le pansement dans une configuration enroulée et encapsulée.

7. Pansement selon l'une quelconque des revendications précédentes, **caractérisé en ce que** les éléments tubulaires (7) ont une géométrie essentiellement cylindrique.
